# EUROPEAN PATENT SPECIFICATION

(11) **EP 1 000 591 B1**
(45) Date of publication and mention of the grant of the patent: **06.04.2005**
(21) Application number: 99309063.8
(22) Date of filing: 15.11.1999
(51) Int. Cl.: A61F 2/06

(54) **Balloon catheter having enhanced stent retention**
Ballonkatheter mit erhöhter Stentretention
Cathéter à ballonnet avec une rétention de stent augmentée

(30) Priority: 16.11.1998 US 193170
(43) Date of publication of application: 17.05.2000
(73) Proprietor: Cordis Corporation, Miami Lakes Florida 33014 (US)
(72) Inventor: Juman, Mohamad Ike, Miami, Florida 33184 (US); Miller, Jay, Miramar, Florida 33025 (US); Play, Edward J., Weston, Florida 33326 (US)
(74) Representative: Mercer, Christopher Paul

(56) References cited:
- EP-A- 0 778 010
- EP-A- 0 855 171
- WO-A-97/21400
- US-A- 4 762 128
- US-A- 5 658 311

## Description

### BACKGROUND AND SUMMARY OF THE INVENTION

### 1. Technical Background:

The present invention relates generally to medical devices, and more particularly to a balloon catheter and stent delivery system.

### 2. Discussion:

Balloon catheters are used in a variety of therapeutic applications, including intravascular catheters for procedures such as angioplasty. Nearly one million angioplasties were performed worldwide in 1997 to treat vascular disease, including coronary, neurological and peripheral blood vessels partially or totally blocked or narrowed by a stenosis. By way of example, the present invention will be described in relation to coronary and peripheral angioplasty treatments. However, it should be understood that the present invention relates to any balloon catheter and stent delivery system having enhanced stent retention, and is not limited to angioplasty.

Most balloon catheters have a relatively long and flexible tubular shaft defining one or more passages or lumens, and an inflatable balloon attached near one end of the shaft. This end of the catheter where the balloon is located is customarily referred to as the "distal" end, while the other end is called the "proximal" end. The balloon is connected to one of the lumens extending through the shaft for the purpose of selectively inflating and deflating the balloon. The other end of this inflation lumen leads to a hub coupling at the other end for connecting the shaft lumens to various equipment. Examples of this type of balloon catheter are shown in U.S. Patent number 5,304,197, entitled "Balloons For Medical Devices And Fabrication Thereof," issued to Pinchuk et al. on April 19th, 1994, and also in U.S. Patent number 5,370,615, entitled "Balloons Catheter For Angioplasty," issued to Johnson on Dec. 6, 1994.

A common treatment method for using such a balloon catheter is to advance the catheter into the body of a patient, by directing the catheter distal end percutaneously through an incision and along a body passage until the balloon is located within the desired site. The term "desired site" refers to the location in the patient's body currently selected for treatment by a health care professional. After the balloon is disposed within the desired site, it can be selectively inflated to press outward on the body passage at relatively high pressure to a relatively constant diameter, in the case of an inelastic or non-compliant balloon material.

This outward pressing of a constriction or narrowing at the desired site in a body passage is intended to partially or completely re-open or dilate that body passageway or lumen, increasing its inner diameter or cross-sectional area. In the case of a blood vessel, this procedure is referred to as angioplasty. The objective of this procedure is to increase the inner diameter or cross-sectional area of the vessel passage or lumen through which blood flows, to encourage greater blood flow through the newly expanded vessel. The narrowing of the body passageway lumen is called a lesion or stenosis, and may be formed of hard plaque or viscous thrombus.

Unfortunately, within approximately six months after angioplasty, the lumen at the angioplasty site may re-close or become narrow again. This phenomenon is called restenosis, and may occur in as many as 30-40% of percutaneous transluminal angioplasty patients. Restenosis may require an additional procedure, such as another angioplasty, drug therapy treatment, or even surgery including bypass graft. It is of course desirable to prevent or limit the occurrence of restenosis, especially since some patients may not be preferred candidates for another interventional treatment.

In an effort to prevent restenosis, short flexible cylinders or scaffolds made of metal or polymers, referred to as a stent, may be permanently implanted into the vessel to hold the lumen open, to reinforce the vessel wall and improve blood flow. The presence of a stent tends to keep the blood vessel open longer, but their use may be limited by various factors, including size and location of the blood vessel, a complicated or tortuous vessel pathway, etc. Also, even a vessel with a stent may eventually develop restenosis.

Some stents are expanded to the proper size by inflating a balloon catheter, referred to as "balloon-expandable" stents, while others are designed to elastically resist compression in a "self-expanding" manner. Both balloon-expandable stents and self-expanding stents are generally crimped or compressed to a diameter during delivery that is smaller than the eventual deployed diameter at the desired site. When positioned at the desired site within the lesion, they are deployed by inflating a balloon or being allowed to self-expand into the desired diameter.

Friction forces may tend to cause a crimped stent to slip in a proximal direction while the catheter system is advanced, or to slip in a distal direction if the physician decides to withdraw the stent without deploying it. It is of course desirable to retain the stent in the proper position, during advancement along a vascular path to the desired site.

In each of EP-A-0855171, WO 97/21400, EP-A-0778010 and US 4,762,128, there is disclosed a balloon catheter stent deployment system of the type set forth in the preamble to claim 1.

Accordingly, it is an object of the present invention to provide balloon catheter systems for enhanced stent position retention during longitudinal movement of the catheter.

In accordance with the present invention, there is provided a balloon catheter stent deployment system as set forth in the accompanying claim 1.

These and various other objects, advantages and features of the invention will become apparent from the following description and claims, when considered in conjunction with the appended drawings.

### BRIEF DESCRIPTION OF THE DRAWINGS

Figure 1 is an external perspective view of a balloon catheter having a stent mounted around the balloon, arranged according to the principles of the present invention;

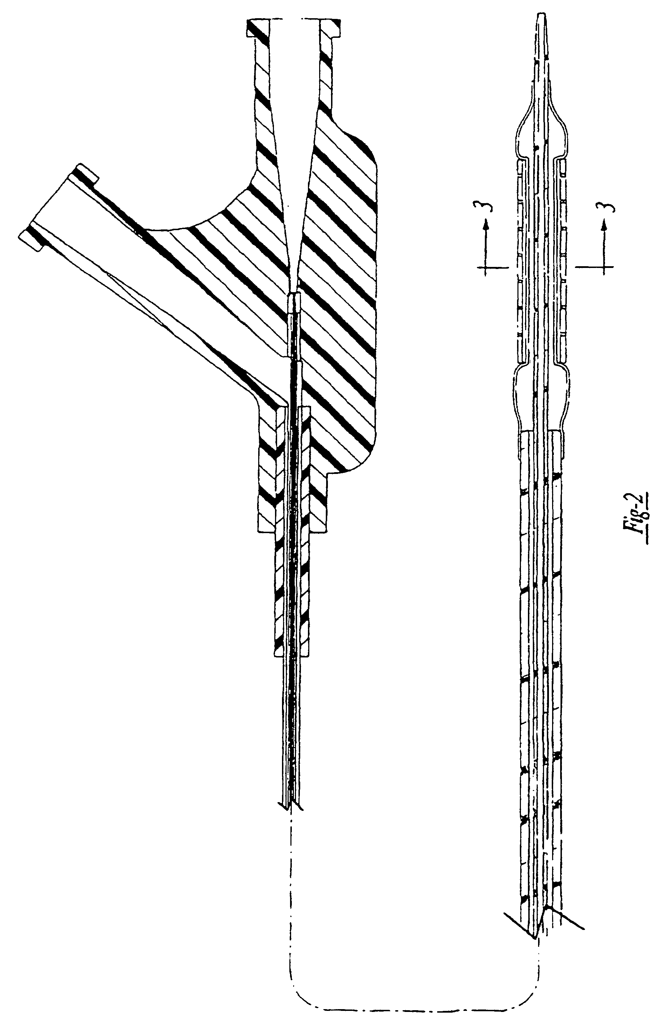

Figure 2 is a longitudinal cross-section view of the balloon catheter and stent of Figure 1;

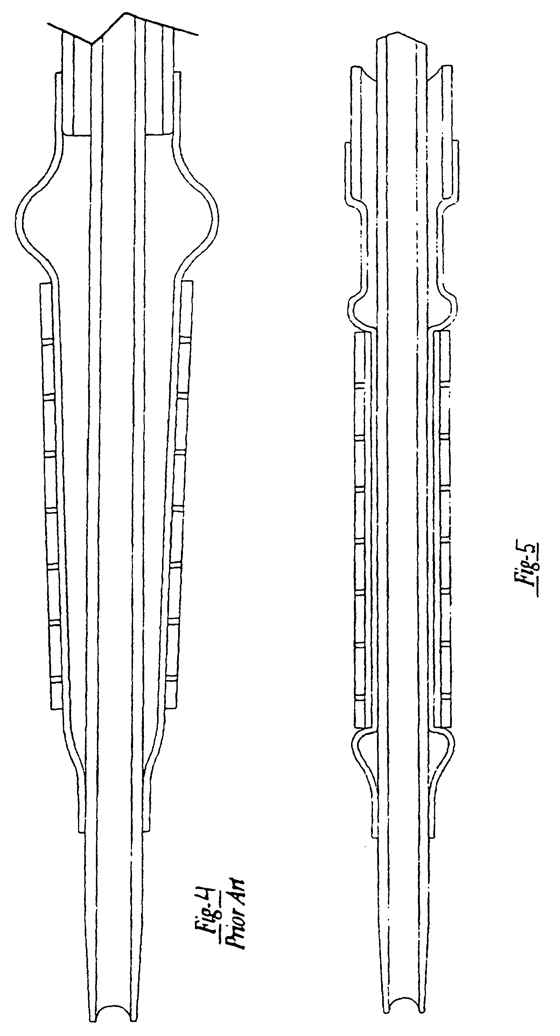

Figure 3 is a transverse cross-section view of the balloon catheter and stent of Figure 2, taken along line 3-3;
Figure 4 is a longitudinal cross-section view of a balloon catheter and stent, according to the prior art;
Figure 5 is a partial longitudinal cross-section view of a deflated balloon catheter and stent, arranged according to the principles of the present invention;
Figure 6 is a partial longitudinal cross-section view of a partially inflated balloon catheter and stent;
Figure 7 is a partial longitudinal cross-section view of a fully inflated balloon catheter and stent; and

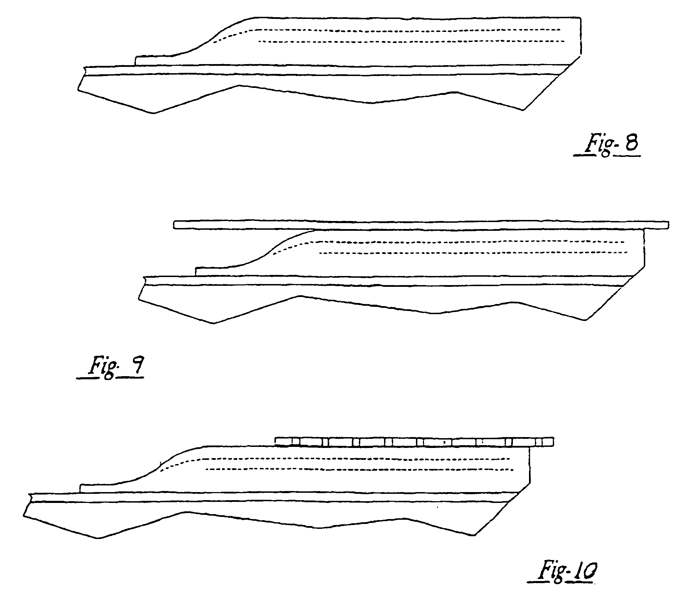

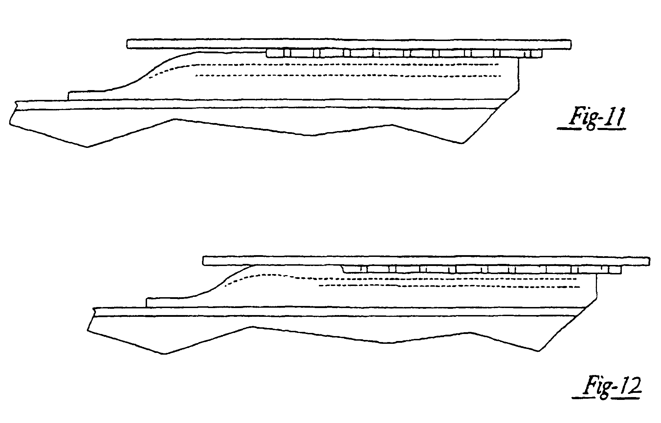

Figures 8-12 illustrate a method for making the balloon catheter stent delivery system of the present invention.

The following description of the preferred embodiments of the present invention is merely illustrative in nature, and as such it does not limit in any way the present invention, its application, or uses. Numerous modifications may be made by those skilled in the art without departing from the true spirit and scope of the invention.

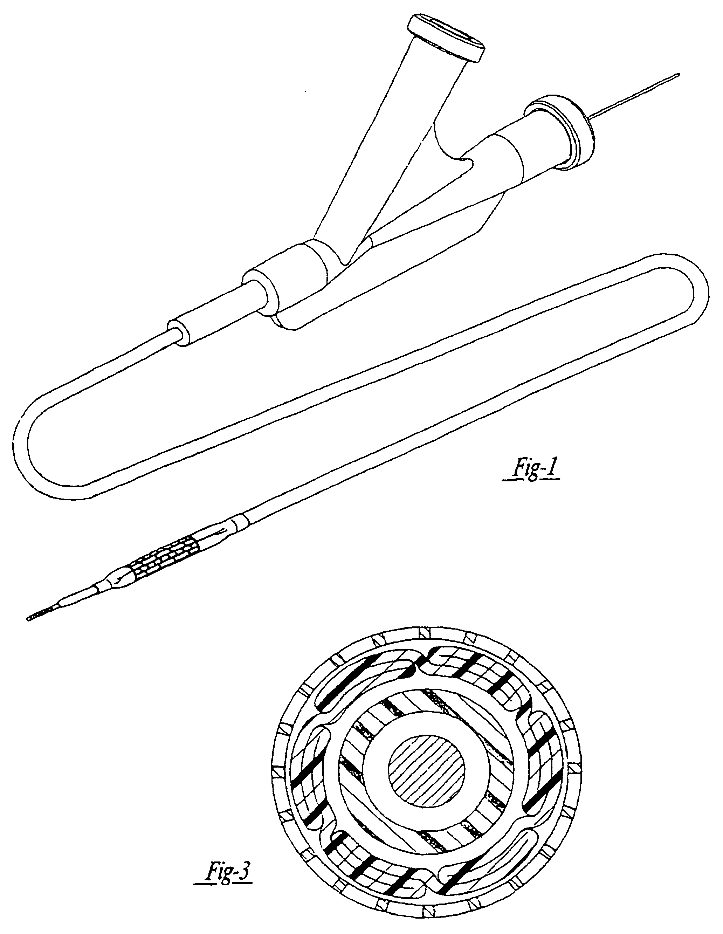

Referring to the drawings, a balloon catheter system is depicted, with one of the preferred embodiments of the present invention being shown generally at 10. The balloon catheter of Figure 1 has an inflatable balloon, a relatively long and flexible tubular shaft, and a hub. The balloon is affixed to the shaft near a distal end of the shaft, and the hub is affixed to the proximal end of the shaft.

The shaft defines one or more passages or lumens extending through the shaft, at least one of which is an inflation lumen connected to the balloon for the purpose of selectively inflating and deflating the balloon. The inflation lumen thus provides fluid communication between the interior of the balloon at the distal end of the inflation lumen, and a hub inflation port having a coupling or luer-lock fitting at the proximal end for connecting the inflation lumen to a source of pressurized inflation fluid (not shown) in the conventional manner.

In the illustrated embodiment, the shaft is constructed of an inner and outer tubular body. The inner body defines a guidewire lumen, while the inflation lumen is defined by the annular space between the inner and outer tubular bodies. The guidewire lumen is adapted to receive an elongated flexible guidewire in a sliding fashion, such that the guidewire and catheter may be advanced or withdrawn independently, or the catheter may be guided along a path selected with the guidewire. The shaft may of course have various configurations instead of this coaxial design, including a single extruded tube defining any suitable number of parallel side-by-side lumens, a proximal shaft portion formed of a metal hypotube, and others.

The proximal hub is affixed to the proximal end of the shaft, and provides an inflation port and a guidewire port, again with a luer-lock fitting or hemostatic valve. Such a valve allows the guidewire to traverse and slide within the guidewire lumen, yet while resisting the loss of blood or other fluids through the guidewire lumen and guidewire port. As shown in the drawings, the inner and outer tubular bodies are securely received within the hub, and surrounded by a tubular strain relief. The hub provides fluid communication between the guidewire lumen and a guidewire coupling, as well as between the annular inflation lumen and the inflation coupling.

A stent of any suitable type or configuration may be provided with the catheter of the present invention, such as the well-known Palmaz-Schatz balloon expandable stent. Various kinds and types of stents are available in the market, and many different currently available stents are acceptable for use in the present invention, as well as new stents which may be developed in the future. The stent depicted in the drawings is a cylindrical metal mesh stent having an initial crimped outer diameter, which may be forcibly expanded by the balloon to a deployed diameter. When deployed in a body passageway of a patient, the stent may be designed to preferably press radially outward to hold the passageway open.

As shown in the drawings, the balloon in its fully inflated profile shape has a cylindrical working portion with an inflated diameter located between a pair of conical end portions, and a pair of proximal and distal legs affixed to the shaft. The balloon in its deflated profile shape preferably has several pleats that are wrapped around the shaft. The balloon material is preferably substantially inelastic, and stretches a relatively small amount under pressures of 5 atmospheres or more. Various different materials may be used, including Nylon, PEEK, Pebax, or a block copolymer thereof.

The novel balloon catheter system of the present invention provides several advantages. Among these advantages is that the balloon has a composite profile shape which varies at different pressures. The balloon initially is in a deflated state and has a deflated profile shape, as specifically illustrated in Figure 5, having a central bed portion with a deflated bed diameter being flanked by a pair of proximal and distal pillows defining deflated pillow diameters that are larger than the deflated bed diameter.

The balloon pillows smoothly taper in proximal and distal directions respectively to proximal and distal legs that are affixed to the shaft. This deflated balloon profile shape thus provides a bed or nest portion for receiving the stent and tending to hold the stent in place, while minimizing friction or adverse contact between the ends of the stent and the blood vessel wall. The present invention thus tends to protect the leading or distal ends of the stent during advancement into the patient's body, and the proximal end of the stent during any withdrawal of the catheter system.

As specifically shown in Figure 6, while the balloon is inflated at intermediate pressures, it will tend to exhibit nested profile shapes similar to the original deflated and nested profile shape of Figure 5. One essential feature of the present invention is the formation of small channels that facilitate fluid communication from the proximal end of the balloon to the distal end, even when the balloon is deflated. The balloon thus tends to inflate more uniformly along its length, such that both proximal and distal balloon pillows inflate at substantially the same times and pressures.

In addition, the present balloon catheter system can be modified to initiate partial inflation of the proximal and distal ends of the stent, to further resist longitudinal motion of the stent during inflation, and to facilitate more effectively fixing the stent in place within the blood vessel, called "tacking" the stent.

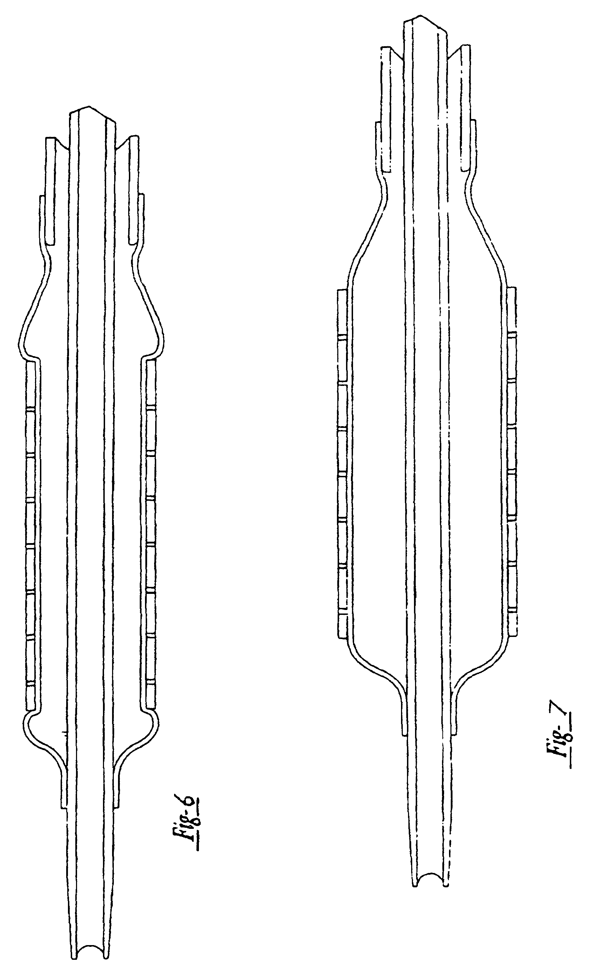

Figure 7 depicts the balloon in its fully inflated profile shape. The stent bed shape disappears, and the balloon profile shape changes or morphs into a different profile shape when inflated at full inflation pressure. This fully inflated shape provides the preferable cylindrical working portion, wherein the portion of the balloon supporting and expanding the stent has an inflated diameter larger than any other portion of the balloon. This feature tends to prevent any part of the balloon from expanding excessively, which might cause local trauma to the blood vessel wall.

Accordingly, the portions of the present balloon reverse positions. The central bed portion initially has a smaller deflated diameter than the proximal and distal pillows, which provides a desirably small outer maximum diameter for ease of insertion. The initial outer maximum diameter is referred to as the "primary profile." In contrast, the central balloon portion expands on full inflation to the largest diameter of the balloon, while the portions that previously formed the balloon pillows expand comparably less. Indeed, the former pillows define the proximal and distal end conical portions of the fully inflated profile shape.

In the deflated shape, the balloon is therefore temporarily reformed into a different shape than what might conventionally result from simply deflating and pleating a previously known balloon. This temporarily reformed shape enhances stent position retention, and yet exhibits the preferable fully inflated shape. The balloon of the present invention also tends to reduce the maximum profile diameter after the balloon is deflated, referred to as the "secondary profile."

Another advantage of the present invention is the absence of any type of physical collar or other retaining device within the balloon, or on the outer balloon surface, or mounted on the balloon catheter shaft, which might undesirably increase the primary and/or secondary profiles of the stent delivery system.

One possible feature of the catheter system is that the balloon may be pleated in a particular pattern when deflated, whereby the central bed portion of the balloon that carries the stent has a greater number of pleats than the proximal and distal balloon pillows.

The balloon catheter system of the present invention may be made using any of the following methods, as well as various modifications that will be apparent to those skilled in the art. The balloon is folded into any suitable or preferable number of longitudinal pleats which are wrapped around a portion of the catheter shaft, either manually or by using a pleating machine.

The balloon is then temporarily held in its pleated condition by slipping a forming tube in the proximal direction onto the pleated balloon, while the assembly is transported to the next processing station. The pleated balloon may be allowed to sit overnight, which may improve its tendency to hold its pleated shape. After the forming tube is removed, a stent is slipped onto the pleated balloon. The stent is then gently crimped or compressed around the balloon, with the pleats intact, to a crimped condition in which the stent has a crimped outer diameter.

The resulting balloon catheter and stent assembly is then placed in a tubular mold, having an internal diameter slightly greater than the crimped outer diameter of the stent. The tubular mold should have a constant inner diameter, to cause the balloon pillows to have the preferred shape and diameter.

The balloon is then pressurized by applying a pressurized gas or fluid to the inflation port and through the inflation lumen. The preferred pressure of the inflation within the tubular mold may slightly exceed the rated burst pressure of the balloon, and the mold will prevent expansion nf the stent while allowing the proximal and distal balloon pillows to form. The pressurized gas or fluid may preferably be dry nitrogen, and the pressure may preferably be maintained for a preselected period of time, such as several minutes.

While mold with the accompanying balloon catheter and stent assembly is held under pressure, they are then held in a hot liquid bath, for several purposes. First, the heat tends to set the stent in place, thus forming the desired proximal and distal pillows. Second, if the balloon is made of Nylon according to one of the preferred embodiments, then the water of the heating bath tends to hydrate the Nylon plasticizer of the balloon material. Of course, a hot air system or any heat source system may also be used. The preferred temperature of the heated water bath is preferably below the permanent deformation temperature of the balloon material, and the time and pressure of this process may be extended to ensure that such a temperature will result in the desired composite shape and temporary reformation of the balloon.

The balloon, stent and mold assembly is then removed from the heated liquid bath, while the pressure is maintained for a period of time. After the pressure is relieved, the mold is then removed, and the balloon and stent assembly may be dried and again heated by applying a hot air gun for a period of time.

Several features of this preferred method of making the balloon catheter stent delivery system of the present invention have some importance to the performance of the resulting product, including the temperatures, pressures, time periods, crimped outer diameter of the stent, the internal diameter of the mold, as well as the thermal characteristics of the balloon, stent and mold.

The particular preferred method described above for making a balloon catheter stent delivery system obviously produces a balloon catheter having an already mounted stent. However, the methods of the present invention may also be used to produce a balloon catheter having the desired enhanced stent retention capability, without incorporating an included stent. Accordingly, the physician may then install and manually crimp any selected stent having the proper dimensions, while yet taking advantage of the enhanced stent position retention of the present invention. Also, this modified method of making the balloon of the present invention may be modified to produce balloon pillows that have a greater initial outer diameter than that of the stent.

Accordingly, a balloon catheter having enhanced stent position retention may be made, without requiring a stent during the process, by a method similar to that described above. During this modified method, the presence of a stent is obviated by replacing it with a 'phantom stent.' One advantage of using a phantom stent is that it costs much less than an actual metal stent. Such a phantom stent may be formed of any suitable plastic material capable of withstanding the temperatures and pressures of the manufacturing method without melting or deforming. Suitable plastic materials for the phantom stent may thus include for example, PTFE or polyethylene.

Since the plastic phantom stent will not crimp in the same way that an actual metal stent does, it must be provided with a longitudinal slit or preferably a spiral cut. The phantom stent may thus be installed onto the pleated balloon, and removed after forming the stent nest with the accompanying pillows, by way of the cut in the plastic material.

When a balloon catheter is made and processed according to the methods of the present invention, and then the phantom stent is removed, the resulting balloon catheter has a stent nest or bed portion which will provide enhanced stent position retention for any stent of suitable dimensions.

Indeed, the size of the resulting balloon pillows may be tailored by carefully selecting the tubular wall thickness of the plastic phantom stent to be greater than the wall thickness of the stent.

It should be understood that an unlimited number of configurations for the present invention could be realized. The foregoing discussion describes merely exemplary embodiments illustrating the principles of the present invention, the scope of which is recited in the following claims. Those skilled in the art will readily recognize from the description, claims, and drawings that numerous changes and modifications can be made without departing from the scope of the invention as defined by the accompanying claims.

## Claims

1. A balloon catheter stent deployment system, comprising:
a balloon catheter with an elongated flexible shaft having proximal and distal ends, a hub affixed to the shaft proximal end and having an inflation port, and an inflatable balloon affixed to the shaft near the shaft distal end; the shaft defining an inflation lumen providing fluid communication of an inflation fluid between the hub inflation port and the balloon,
such that the balloon is adapted for selective inflation from a deflated state to an inflated state, as well as later deflation; the balloon being formed of a substantially inelastic material; the balloon having a cylindrical working portion with an inflated cross-sectional area located between a pair of conical portions and a pair of leg portions when the balloon is in the inflated state; the balloon being initially in the deflated state having multiple longitudinal pleats;
an expandable tubular mesh stent defining a tubular wall thickness, mounted around the balloon and being crimped in an initial state to an initial outer diameter defined by an outer surface of the stent; the stent having substantially no tendency to self-expand absent an expansive force caused by inflating the balloon;
wherein the balloon in its deflated state defines a first and second pillow located immediately proximal and distal of the stent; each balloon pillow in an initial deflated state having an initial outer diameter equal to at least the initial outer diameter of the stent, thereby tending to frictionally retain the stent in an initial longitudinal position while the catheter system is advanced or withdrawn along a vascular path; such that the balloon defines an indented bed shape for the stent in the deflated state; and
wherein the balloon is adapted to inflate and expand the stent to a deployed larger diameter, whereby the balloon forms said cylindrical working portion at above a preselected transition pressure, such that the cylindrical working portion and the portions defining the balloon pillows in the deflated state have substantially the same diameter in the inflated state; thereby causing said indented bed shape to substantially disappear, **characterised by** a plurality of small channels formed in the balloon that facilitate fluid communication from the proximal end of the balloon to the distal end of the balloon, even when the balloon is in the deflated state, such that the balloon tends to inflate at its proximal and distal ends in a substantially simultaneous manner.

2. The balloon catheter stent deployment system of Claim 1, wherein the initial outer diameter of the first and second balloon pillows in the initial deflated state are greater than the initial outer diameter of the stent, such that the pillows provide an enhanced tendency to retain the stent in its initial longitudinal position, and provide enhanced protection of the stent.

3. The balloon catheter stent deployment system of Claim 1, wherein said stent is formed of an integral unitary metal cylinder having a plurality of apertures for allowing the stent to expand from the initial diameter to the deployed diameter.

4. The balloon catheter stent deployment system of Claim 1, wherein the stent is formed of one or more wires arranged into an integral non-unitary metal cylinder having one or more attachments between selected portions of the wires.

5. The balloon catheter stent deployment system of Claim 1, wherein said balloon is formed of a material selected from the group of Nylon, PEEK, Pebax, or a block copolymer thereof.

6. The balloon catheter stent deployment system of Claim 1, further comprising a guidewire lumen adapted to slidably receive an elongated flexible guidewire; the guidewire lumen extending continuously from a distal guidewire port defined by the catheter distal of the balloon to a proximal guidewire port near the proximal end of the shaft, in an over-the-wire configuration.

7. The balloon catheter stent deployment system of Claim 1, further comprising a guidewire lumen adapted to slidably receive an elongated flexible guidewire; the guidewire lumen extending continuously from a distal guidewire port defined by the catheter distal of the balloon to a proximal guidewire port at a point nearer to the catheter distal end than the catheter proximal end, in a rapid exchange configuration.

8. The balloon catheter stent deployment system of Claim 1, wherein said pillows in the deflated state are adapted to protect the proximal and distal ends of the stent and reduce the possibility of undesirable contact between the proximal and distal ends of the stent and a sidewall of the vascular path.

9. A balloon catheter of any preceding Claim, wherein the balloon pillows in the deflated shape expand to form some of the cylindrical working portion, as well as the conical portions, in the inflated shape.

10. A balloon catheter of any preceding Claim, wherein the plurality of folded pleats, wrapped around the shaft; the pleats tending to expand and unfold in the inflated shape.

11. A balloon catheter of any preceding Claim, wherein a portion of the shaft that is within the balloon has a substantially constant outer cross-section which is substantially free of protrusions, and the balloon material having a substantially constant wall thickness; thereby minimizing the secondary profile, defined as the maximum outer diameter of any portion of the balloon following inflation and deflation.

## Patentansprüche

1. Ballonkatheterstententfaltungssystem umfassend:
einen Ballonkatheter mit einem verlängerten flexiblen Schaft mit einem proximalen und einem distalen Ende, einem Sitz, der mit dem proximalen Ende des Schaftes verbunden ist und eine Aufblasöffnung aufweist, und einem aufblasbaren Ballon, der an dem Schaft nahe dem distalen Ende des Schaftes angebracht ist; wobei der Schaft einen Aufblashohlraum definiert, der eine Fluidverbindung eines Aufblasfluids zwischen der Sitzaufblasöffnung und dem Ballon bereitstellt,
so dass der Ballon angepasst ist für das selektive Aufblasen aus einem entleerten Zustand in einen aufgeblasenen Zustand, ebenso wie spätere Entleerung; der Ballon aus einem im wesentlichen unelastischen Material ausgebildet ist, der Ballon einen zylindrischen Arbeitsabschnitt mit einer aufgeblasenen Querschnittsfläche aufweist, die zwischen einem Paar konischer Abschnitte und einem Paar Schenkelabschnitten angeordnet ist, wenn sich der Ballon in seinem aufgeblasenen Zustand befindet; der Ballon sich anfangs in dem entleerten Zustand mit einer Vielzahl von Falten in Längsrichtung befindet;
einen expandierbaren röhrenförmigen Gitterstent, der eine röhrenförmige Wandstärke definiert, angeordnet um den Ballon und in einem anfänglichen Zustand auf einen anfänglichen äußeren Durchmesser gefaltet, der durch eine äußere Oberfläche des Stents definiert ist; der Stent im wesentlichen keine Neigung aufweist, ohne expansive Kraft, die durch Aufblasen des Ballons verursacht wird, selbst zu expandieren;
wobei der Ballon in seinem entleerten Zustand ein erstes und zweites Kissen definiert, das unmittelbar proximal und distal des Stents angeordnet ist; jedes Ballonkissen in einem anfänglichen entleerten Zustand einen anfänglichen äußeren Durchmesser aufweist, der gleich wenigstens dem anfänglichen äußeren Durchmessers des Stents ist, wodurch er dazu neigt, den Stent durch Reibung in einer anfänglichen Längsposition zurückzuhalten, während das Kathetersystem entlang einem vaskularen Weg nach vorne geschoben oder zurückgezogen wird; so dass der Ballon eine eingebuchtete Bettform für den Stent im entleerten Zustand definiert; und
wobei der Ballon angepasst ist, um den Stent aufzublasen und auf einen entfalteten größeren Durchmesser zu expandieren, wobei der Ballon den zylindrischen Arbeitsabschnitt oberhalb eines zuvor gewählten Übergangsdruckes ausbildet, so dass der zylindrische Arbeitsabschnitt und die die Ballonkissen definierenden Abschnitte im entleerten Zustand im wesentlichen den gleichen Durchmesser im aufgeblasenen Zustand aufweisen; wodurch verursacht wird, dass die eingebuchtete Bettform im wesentlichen verschwindet, **dadurch gekennzeichnet, dass** in dem Ballon eine Vielzahl kleiner Kanäle ausgebildet sind, die die Fluidverbindung von dem proximalen Ende des Ballons zu dem distalen Ende des Ballons erleichtern, selbst wenn sich der Ballon in dem entleerten Zustand befindet, so dass der Ballon dazu neigt, sich im wesentlichen gleichzeitig an seinem proximalen und distalen Ende aufzublasen.

2. Ballonkatheterstententfaltungssystem nach Anspruch 1, wobei der anfängliche Außendurchmesser des ersten und zweiten Ballonkissens in dem anfänglichen entleerten Zustand größer ist als der anfängliche äußere Durchmesser des Stents, so dass die Kissen eine verstärkte Neigung aufweisen, den Stent in seiner anfänglichen Längsposition zurückzuhalten und einen verbesserten Schutz des Stents bereitstellen.

3. Ballonkatheterstententfaltungssystem nach Anspruch 1, wobei der Stent aus einem integralen einstückigen Metallzylinder mit einer Vielzahl von Öffnungen ausgebildet ist, um zu erlauben, dass sich der Stent von dem anfänglichen Durchmesser auf den entfalteten Durchmesser expandiert.

4. Ballonkatheterstententfaltungssystem nach Anspruch 1, wobei der Stent aus einem oder mehreren Drähten ausgebildet ist, die zu einem integralen nicht-einstückigen Metallzylinder angeordnet sind, der ein oder mehrere Befestigungen zwischen ausgewählten Abschnitten der Drähte aufweist.

5. Ballonkatheterstententfaltungssystem nach Anspruch 1, wobei der Ballon aus einem Material ausgebildet ist, das aus der Gruppe Nylon, PEEK, Pebax, oder einem Blockcopolymer davon ausgewählt ist.

6. Ballonkatheterstententfaltungssystem von Anspruch 1, weiter umfassend einen Führungsdrahthohlraum, der angepasst ist, um einen verlängerten flexiblen Führungsdraht verschiebbar aufzunehmen; wobei sich der Führungsdrahthohlraum kontinuierlich von einer distalen Führungsdrahtöffnung, die durch den Katheter distal des Ballons definiert ist, zu einer proximalen Führungsdrahtöffnung nahe dem proximalen Ende des Schaftes erstreckt, in einer über-dem-Draht-Konfiguration.

7. Ballonkatheterstententfaltungssystem nach Anspruch 1, weiter umfassend einen Führungsdrahthohlraum, der angepasst ist, um einen verlängerten flexiblen Führungsdraht verschiebbar aufzunehmen; wobei sich der Führungsdrahthohlraum kontinuierlich von einer distalen Führungsdrahtöffnung, die durch den Katheter distal des Ballons definiert ist, zu einer proximalen Führungsdrahtöffnung an einem Punkt näher zu dem distalen Ende des Katheters als das proximale Ende des Katheters erstreckt, in einer Schnellwechselkonfiguration.

8. Ballonkatheterstententfaltungssystem nach Anspruch 1, wobei die Kissen in dem entleerten Zustand angepasst sind, um das proximale und distale Ende des Stents zu schützen und die Möglichkeit eines unerwünschten Kontaktes zwischen dem proximalen und distalen Ende des Stents und einer Seitenwand des vaskularen Weges zu verringern.

9. Ballonkatheterstententfaltungssystem nach einem der vorangehenden Ansprüche, wobei sich die Ballonkissen in der entleerten Form expandieren, um einen Teil des zylindrischen Arbeitsabschnittes auszubilden, ebenso wie die konischen Abschnitte in der aufgeblasenen Form.

10. Ballonkatheterstententfaltungssystem nach einem der vorangehenden Ansprüche, wobei die Vielzahl von gefalteten Falten um den Schaft gewickelt sind, wobei die Falten dazu neigen, sich in der aufgeblasenen Form zu expandieren und zu entfalten.

11. Ballonkatheter nach einem der vorangehenden Ansprüche, wobei ein Abschnitt des Schaftes, der innerhalb des Ballons ist, einen im wesentlichen konstanten äußeren Querschnitt aufweist, der im wesentlichen frei von Vorwölbungen ist, und das Ballonmaterial eine im wesentlichen konstante Wandstärke aufweist; wodurch das Sekundärprofil minimiert wird, das als der maximale Außendurchmesser eines jeden Abschnittes des Ballons nach Aufblasen und Entleeren definiert ist.

## Revendications

1. Système de déploiement d'endoprothèse vasculaire à cathéter à ballon, comportant :
un cathéter à ballon ayant un arbre souple allongé ayant des extrémités proximale et distale, un moyeu fixé sur l'extrémité proximale de l'arbre et ayant un orifice de gonflage, et un ballon gonflable fixé sur l'arbre à proximité de l'extrémité distale de l'arbre, l'arbre définissant une lumière de gonflage fournissant une communication de fluide d'un fluide de gonflage entre l'orifice de gonflage de moyeu et le ballon,
de telle sorte que le ballon est adapté pour un gonflage sélectif à partir d'un état dégonflé vers un état gonflé, de même que pour un dégonflage ultérieur, le ballon étant formé en un matériau sensiblement non-élastique, le ballon ayant une partie de travail cylindrique ayant une coupe transversale gonflée positionnée entre une paire de parties coniques, et une paire de parties formant patte lorsque le ballon est dans l'état gonflé, le ballon initialement dans l'état dégonflé, ayant de multiples plis longitudinaux,
une endoprothèse vasculaire en treillis tubulaire extensible définissant une épaisseur de paroi tubulaire, montée autour du ballon et sertie dans un état initial sur un diamètre extérieur initial défini par une surface extérieure de l'endoprothèse vasculaire, l'endoprothèse vasculaire n'ayant sensiblement pas tendance à se déployer automatiquement en l'absence d'une force de déploiement provoquée par un gonflage du ballon,
dans lequel le ballon, dans son état dégonflé, définit des premier et second coussinets positionnés immédiatement proximal et distal par rapport à l'endoprothèse vasculaire, chaque coussinet de ballon dans un état dégonflé initial ayant un diamètre extérieur initial au moins égal au diamètre extérieur initial de l'endoprothèse vasculaire, de manière à avoir tendance à retenir par frottement l'endoprothèse vasculaire dans une position longitudinale initiale tandis que le système de cathéter est avancé ou retiré le long d'un trajet vasculaire, de telle sorte que le ballon définit une forme de lit denté pour l'endoprothèse vasculaire dans l'état dégonflé, et
dans lequel le ballon est adapté pour gonfler et déployer l'endoprothèse vasculaire jusqu'à un diamètre déployé supérieur, de sorte que le ballon forme ladite partie de travail cylindrique au-dessus d'une pression de transition présélectionnée, de telle sorte que la partie de travail cylindrique et les parties définissant les coussinets de ballon dans l'état dégonflé ont sensiblement le même diamètre dans l'état gonflé, en amenant ainsi ladite forme de lit denté à disparaître sensiblement, **caractérisé par**
une pluralité de petits canaux formés dans le ballon, qui facilitent la communication de fluide à partir de l'extrémité proximale du ballon vers l'extrémité distale du ballon, même lorsque le ballon est dans l'état dégonflé, de telle sorte que le ballon tend à se gonfler au niveau de ses extrémités proximale et distale d'une manière sensiblement simultanée.

2. Système de déploiement d'endoprothèse vasculaire à cathéter à ballon selon la revendication 1, dans lequel les diamètres extérieurs initiaux des premier et second coussinets de ballon, dans l'état dégonflé, sont supérieurs au diamètre extérieur initial de l'endoprothèse vasculaire, de telle sorte que les coussinets fournissent une tendance améliorée à retenir l'endoprothèse vasculaire dans sa position longitudinale initiale, et fournissent une protection améliorée de l'endoprothèse vasculaire.

3. Système de déploiement d'endoprothèse vasculaire à cathéter à ballon selon la revendication 1, dans lequel ladite endoprothèse vasculaire est formée d'un cylindre de métal unitaire en un seul bloc ayant une pluralité d'ouvertures pour permettre à l'endoprothèse vasculaire de se déployer à partir du diamètre initial vers le diamètre déployé.

4. Système de déploiement d'endoprothèse vasculaire à cathéter à ballon selon la revendication 1, dans lequel l'endoprothèse vasculaire est constituée d'un ou de plusieurs fils agencés dans un cylindre de métal non-unitaire en un seul bloc ayant une ou plusieurs fixations entre des parties sélectionnées des fils.

5. Système de déploiement d'endoprothèse vasculaire à cathéter à ballon selon la revendication 1, dans lequel ledit ballon est constitué d'un matériau sélectionné dans le groupe comportant Nylon (nom commercial déposé), PEEK (nom commercial déposé), Pebax (nom commercial déposé) ou un copolymère bloc de ceux-ci.

6. Système de déploiement d'endoprothèse vasculaire à cathéter à ballon selon la revendication 1, comportant en outre une lumière de fil de guidage adapté pour recevoir de manière coulissante un fil de guidage souple allongé, la lumière de fil de guidage s'étendant en continu à partir d'un orifice de fil de guidage distal défini par l'extrémité distale du ballon vers un orifice de fil de guidage proximal situé à proximité de l'extrémité proximale de l'arbre, dans une configuration au-dessus du fil.

7. Système de déploiement d'endoprothèse vasculaire à cathéter à ballon selon la revendication 1, comportant en outre une lumière de fil de guidage adapté pour recevoir de manière coulissante un fil de guidage souple allongé, la lumière de fil de guidage s'étendant en continu à partir d'un orifice de fil de guidage distal défini par l'extrémité distale du ballon jusqu'à un orifice de fil de guidage proximal situé au niveau d'un point plus proche de l'extrémité distale du cathéter que de l'extrémité proximale du cathéter, selon une configuration d'échange rapide.

8. Système de déploiement d'endoprothèse vasculaire à cathéter à ballon selon la revendication 1, dans lequel lesdits coussinets, dans l'état dégonflé, sont adaptés pour protéger les extrémités proximale et distale de l'endoprothèse vasculaire, et pour réduire la possibilité d'un contact non-souhaitable entre les extrémités proximale et distale de l'endoprothèse vasculaire et une paroi latérale du trajet vasculaire.

9. Cathéter à ballon selon l'une quelconque des revendications précédentes, dans lequel les coussinets de ballon, dans la forme dégonflée, se gonflent pour former une certaine portion de la partie de travail cylindrique, de même que des parties coniques, dans l'état dégonflé.

10. Cathéter à ballon selon l'une quelconque des revendications précédentes, dans lequel il y a une pluralité de plis pliés, enveloppés autour de l'arbre, les plis ayant tendance à se déployer et à se déplier dans la forme gonflée.

11. Cathéter à ballon selon l'une quelconque des revendications précédentes, dans lequel une partie de l'arbre qui est dans le ballon a une coupe transversale extérieure sensiblement constante qui est sensiblement libre de saillie, et le matériau de ballon ayant une épaisseur de paroi sensiblement constante, en minimisant ainsi le profil secondaire défini comme le diamètre extérieur maximum d'une partie quelconque du ballon à la suite d'un gonflage et d'un dégonflage.
